# EUROPÄISCHE PATENTSCHRIFT

(11) **EP 0 875 761 B1**
(45) Veröffentlichungstag und Bekanntmachung des Hinweises auf die Patenterteilung: **14.03.2001**
(21) Anmeldenummer: 98106191.4
(22) Anmeldetag: 04.04.1998
(51) Int. Cl.: G01N 33/68, G01N 33/569

(54) **Immunoassay zur Aviditätsbestimmung von Immunglobulinen**
Immunoassay for the determination of immunoglobulin avidity
Dosage immunologique pour la détermination de l'avidite d'immunoglobulines

(30) Priorität: 02.05.1997 DE 19718361
(43) Veröffentlichungstag der Anmeldung: 04.11.1998
(73) Patentinhaber: Dade Behring Marburg GmbH, 35001 Marburg (DE)
(72) Erfinder: Zens, Wolfgang Dr., 35041 Marburg (DE)

(56) Entgegenhaltungen:
- WO-A-90/02202
- WO-A-97/09619
- US-A- 5 679 537
- PATENT ABSTRACTS OF JAPAN vol. 012, no. 088 (P-678), 23. März 1988 & JP 62 220865 A (YATORON:KK), 29. September 1987

## Beschreibung

Die vorliegende Erfindung betrifft ein Verfahren sowie ein Diagnostikum zum qualitativen oder quantitativen Nachweis von Antikörpern und zur Bestimmung von deren Avidität. Dadurch wird die Diagnose der frühen Phase viraler, bakterieller oder parasitärer Infektionen ermöglicht. Das erfindungsgemäße Diagnostikum ist in besonderer Weise für die automatisierte Bearbeitung in großen Analyselaboratorien geeignet.

Zum Nachweis von Immunglobulinen in Serum- und Plasmaproben zu medizinisch-diagnostischen Zwecken werden häufig Immunoassays aufgrund ihrer besonders guten Spezifität und Sensitivität eingesetzt. Zudem zeichnen sich Immunoassays durch eine einfache Handhabung aus.

Handelt es sich um akute Infektionen, stützt sich die Diagnose hauptsächlich auf den Nachweis von IgM-Antikörpern. In der Laborpraxis treten aber immer wieder Fälle auf, bei denen eine sichere Unterscheidung zwischen frischer, primärer und abgelaufener oder reaktivierter Infektion ohne zusätzliche Untersuchungen nicht möglich ist. Es ist aber bekannt, daß sich beispielsweise durch zusätzliche Messung der Avidität von IgG-Antikörpern, die für bestimmte Viren, Bakterien oder Parasiten spezifisch sind, serologische Konstellationen, die ohne diese zusätzliche Messung keine oder keine hinreichend genaue Aussage über den Infektionszeitpunkt erlauben, beurteilen lassen.

Aviditätsbestimmungen von Immunglobulinen werden in verschiedenen Testsystemen, beispielsweise in proteindenaturierenden Immunoassays durchgeführt, die dem Fachmann unter anderem aus den folgenden Publikationen bekannt sind: J. Schubert et al. (1996), J. Lab. Med. 20 (12): 713-717; J. J. Gray (1995), J. Virol. Methods 52: 95-104; J. Polanec et al. (1994), J. Clin. Lab. Analysis 8: 16-21; H. O. Kangro et al. (1991), J. Med. Virol. 33: 100-105. Gebräuchliche denaturierende Substanzen sind beispielsweise Harnstoff (Urea), Diethylamine, Guanidine, Thiocyanate.

Die Aviditätsbestimmung ist in der Diagnostik bei viralen und nicht-viralen Infektionserregern, beispielsweise bei Epstein Barr Virus (EBV), Rötelnvirus, Zytomegalievirus (CMV), Hantavirus, Parvovirus B19, Varicella-Zoster-Virus (VZV), Humanem Herpesvirus Typ 6 (HHV-6), Hepatitis C-Virus (HCV), Respiratory Syncycial Virus (RSV), Herpes Simplex Virus Typ 1 oder 2 (HSV-1/ -2) und Toxoplasma gondii, bereits erfolgreich eingesetzt worden. Dennoch setzt sich diese Methode in Analyselaboratorien bedingt durch unter anderem technische Probleme nicht durch. Drei Gründe sind dafür im wesentlichen verantwortlich:
1. Die meist verwendete Substanz Harnstoff ist in der von den Autoren empfohlenen Konzentration am Kristallisationspunkt, was zur Folge hat, daß sämtliche Pipettierspitzen und Schläuche von Geräten zur automatischen oder teilautomatischen Bearbeitung von Immunoassays (Immunoassay-Prozessoren) häufig verstopfen.
2. Verwendet man Harnstoff-Lösungen geringerer Molarität (beispielsweise 5 bis 6 M), reduziert sich der Aussagewert der Methode erheblich.
3. Der in der Literatur angegebene grenzwertige bzw. nicht auswertbare Bereich der Methode ist zu groß.

Es bestand somit ein Bedürfnis, die bekannten Verfahren zur Aviditätsbestimmung von Antikörpern zu verbessern. Insbesondere war die Anwendbarkeit auf automatisierten Immunoassay-Prozessoren verbesserungsbedürftig.

Überraschenderweise wurde im Rahmen vorliegender Erfindung gefunden, daß die vorstehend beschriebenen Probleme dadurch gelöst werden können, daß die bisher nicht in proteindenaturierenden Immunoassays verwendete Substanz Harnstoff-Wasserstoffperoxid (beispielsweise erhältlich von der Carl Roth GmbH + Co Karlsruhe: Artikel-Nummer 7641.1) als proteindenaturierendes Reagenz eingesetzt werden kann. Diese Substanz hat überraschenderweise die Eigenschaft, in einem proteindenaturierenden Immunoassay bereits in einer niedrigen molaren Konzentration (ungefähr 2,5 bis 6,5 M) so wirksam zu sein; wie eine hochmolare (ungefähr 6 bis 8 M) Harnstofflösung.Die vorliegende Erfindung betrifft daher ein Verfahren zum qualitativen oder quantitativen Nachweis eines Antikörpers, worin dieser Antikörper mit dem Antigen, gegen welches er gerichtet ist, in Kontakt gebracht wird, so daß sich Immunkomplexe bilden können, und worin der Reaktionsansatz mit einem proteindenaturierenden Agens in Kontakt gebracht wird, welches Immunkomplexe enthaltend Antikörper niedriger Avidität destabilisiert, während Immunkomplexe enthaltend Antikörper höherer Avidität weitgehend erhalten bleiben, und worin das Ausmaß der Bindung des Antikörpers mit dem Antigen nach einem dem Fachmann bekannten Verfahren bestimmt wird, dadurch gekennzeichnet, daß das proteindenaturierende Agens Harnstoff-Wasserstoffperoxid ist.

Die vorliegende Erfindung betrifft außerdem ein Verfahren zur Bestimmung der Avidität eines Antikörpers, worin der Antikörper in einem ersten und einem zweiten Ansatz unabhängig voneinander mit dem Antigen, gegen welches der Antikörper gerichtet ist, in Kontakt gebracht wird; so daß sich Immunkomplexe bilden können, und worin einer der beiden Ansätze mit einem proteindenaturierenden Agens in Kontakt gebracht wird, welches Immunkomplexe enthaltend Antikörper niedriger Avidität destabilisiert, während Immunkomplexe enthaltend Antikörper höherer Avidität weitgehend erhalten bleiben, und worin das Ausmaß der Bindung des Antikörpers mit dem Antigen in beiden Proben unabhängig voneinander nach einem dem Fachmann bekannten Verfahren bestimmt wird, und wobei sich die Avidität des Antikörpers aus dem Verhältnis des Ausmaßes der Antigen-Antikörperbindungen des ersten und des zweiten Ansatzes ergibt, dadurch gekennzeichnet, daß das proteindenaturierende Agens Harnstoff-Wasserstoffperoxid ist.

Bevorzugt wird ein solches Verfahren, worin das Antigen in festphasengebundener Form mit dem Antikörper in Kontakt gebracht und anschlieβend mit einer harnstoff-wasserstoffperoxidhaltigen Pufferlösung gewaschen wird. Beispielsweise zählen der Enzymimmuntest, Radioimmuntest, Western Blot oder Immunfluoreszenztest zu solchen bevorzugten Verfahren. Dem Fachmann sind jedoch weitere Immuntestsysteme bekannt, die mittels vorliegender Beschreibung ohne weiteres erfindungsgemäß unter Verwendung von Harnstoff-Wasserstoffperoxid ausgeführt werden können.

Erfindungsgemäß sind die obengenannten Verfahren insbesondere zum Nachweis diagnostisch relevanter Antikörper in Körperflüssigkeiten geeignet. Solche Antikörper können gegen Viren, Bakterien oder Parasiten gerichtet sein, wie beispielsweise EBV, Rötelnvirus, CMV, Hantavirus, Parvovirus B19, VZV, HHV 6, HBV, HCV, HIV, RSV, HSV-1, HSV-2 oder Toxoplasma gondii.

Die Aviditätsbestimmung kann vorteilhafterweise mit kommerziell erhältlichen Immunoassays (beispielsweise ELISA) teil- oder vollautomatisch durchgeführt werden. Ein weiterer Vorteil vorliegender Erfindung ist die sehr gute Ergebnisinterpretierbarkeit.

Das erfindungsgemäße Verfahren zur Aviditätdsbestimmung von Antikörpern eignet sich in besonderer Weise zur Differenzierung frischer (d.h. erst kürzlich erfolgter) Infektionen von älteren (d.h. länger zurückliegenden) Infektionen. Insbesondere betrifft die vorliegende Erfindung Verfahren zum Nachweis einer akuten oder kürzlich abgelaufenen Rötelnvirusinfektion, CMV-Erstinfektion, EBV-Erstinfektion, HSV-Erstinfektion oder Toxoplasma gondii-Infektion.

Die vorliegende Erfindung betrifft außerdem Diagnostika (diagnostische Reagenzien, Testbestecke), die geeignet sind, die beschriebenen erfindungsgemäßen Verfahren anzuwenden. Die Herstellung solcher Diagnostika erfolgt in dem Fachmann an sich bekannter Weise, basierend auf der vorliegenden Beschreibung der Erfindung.

### Herstellung der erfindungsgemäßen Harnstoff-Wasserstoffperoxid Lösung

Als Verdünnungslösung für proteindenaturierende Substanzen wird häufig die Waschlösung aus kommerziell erhältlichen ELISA Testkits verwendet. In den folgenden Beispielen wurde exemplarisch die sogenannte "Waschlösung POD" (Best. Nr. OSEW, Behring Diagnostics GmbH, Marburg, Deutschland) verwendet. Allerdings hätte auch jede andere prinzipiell zum Waschen eines ELISA geeignete Waschlösung verwendet werden können. Der Anwendungsbereich von Harnstoff-Wasserstoffperoxid liegt zwischen 2,5 mol/l und 6,5 mol/l, bevorzugt zwischen 4,5 und 6,0 mol/l. Die optimierte Konzentration liegt bei 5,3 mol/l. Denkbar ist der Einsatz von Harnstoff-Wasserstoffperoxid auch in anderen bei der Durchführung von Immunoassays verwendeten Lösungen, beispielsweise in Probenverdünnungspuffern.

### Durchführung des Immunoassays

Mit der oben genannten erfindungsgemäßen Harnstoff-Wasserstoffperoxid Lösung wird nach gängiger Methode ein Immunoassay, beispielsweise ein ELISA durchgeführt.

Die Modifikation der erfindungsgemäßen Methode gegenüber bekannten Verfahren besteht darin, daß nach der Probeninkubation zweimal mit einem an die Methode angepaßten Volumen (im auf Mikrotitrationsplatten basierenden ELISA beispielsweise ca. 0,3 ml) Harnstoff-wasserstoffperoxidhaltiger Waschlösung gewaschen und anschließend beispielsweise zweimal mit jeweils dem gleichen Volumen Waschlösung nachgewaschen wird. Die bei den Automaten vom Hersteller vorgesehene Verweilzeit der Waschlösung bedarf nicht zwingend einer Änderung.

Die Testdurchführung erfolgt - falls automatische Abarbeitung gewünscht wird - an einem ELISA-Prozessor (erhältlich beispielsweise von der Behring Diagnostics GmbH).

In den nachfolgenden Beispielen wurden ELISAs der Behring Diagnostics GmbH verwendet und modifiziert. Als Waschlösung diente die "Waschlösung POD". Diese Beispiele dienen der weiteren Erläuterung der Erfindung, ohne sie jedoch in irgendeiner Weise zu beschränken.

### Beispiele

### Beispiel 1:

Zur Optimierung der ELISA-Aviditätsbestimmung wurde zunächst der Harnstoff- und der Harnstoff-Wasserstoffperoxid-Waschschritt im Vergleich bei Seren mit frischer bzw. alter Röteln-Infektion mit verschiedenen Reagenzmolaritäten durchgeführt.

### Ergebnis:

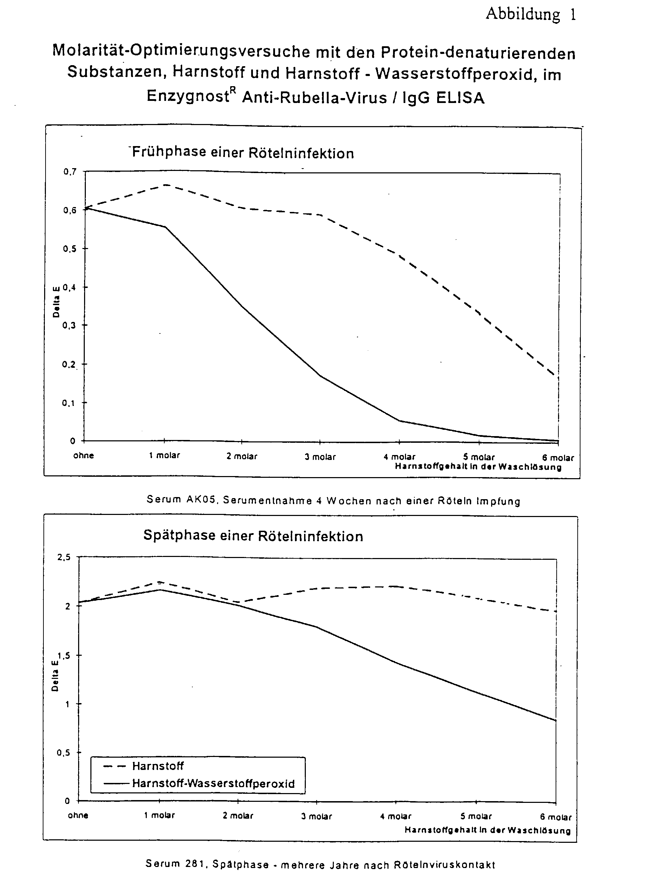

Abbildung 1 zeigt, daß die erfindungsgemäße Harnstoff-Wasserstoffperoxid Lösung in einem Konzentrationsbereich von 2,5 bis 6,5 mol/l die beste Trennung zwischen den beiden Serengruppen erzielt.

### Beispiel 2:

Zur Überprüfung der Aussagefähigkeit des erfindungsgemäßen Verfahrens mit Harnstoff-Wasserstoffperoxid im Vergleich mit der konventionellen Methode gemäß dem Stand der Technik wurde eine Röteln-Serokonversion herangezogen.

### Ergebnis:

Siehe Tabelle 1.- Die ELISA-Aviditätsbestimmung wurde gemäß dem erfindungsgemäßen Verfahren wie folgt ausgewertet: kleiner oder gleich 15 % = akute Infektion, 15 bis 20 % = Toleranzbereich der akuten Infektion, größer oder gleich 20 % = zurückliegende Infektion.

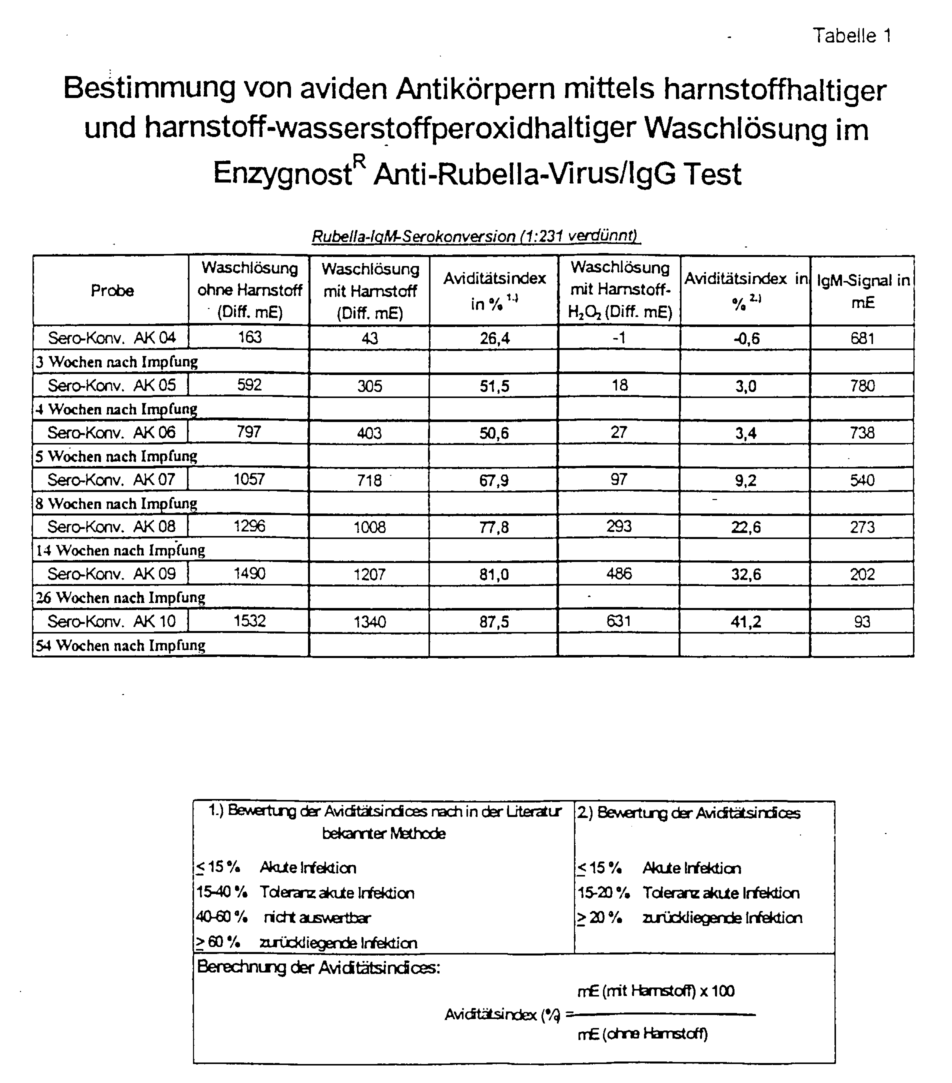

### Beispiel 3:

In einem gesichert Röteln-IgM-positiven Serumkollektiv soll vergleichend zu einem gesichert Röteln-IgM-negativen Serumkollektiv die Trennschärfe der Methode gezeigt werden.

### Ergebnis:

Siehe Tabelle 2. Die Hauptmenge der Seren aus der frühen Phase der Rötelninfektion hat einen Aviditätsindex von kleiner als ungefähr 5%, hingegen haben die meisten Rötelnseren, stammend aus der Spätphase der Infektion, einen durchschnittlichen Aviditätsindex von etwa 50 %.

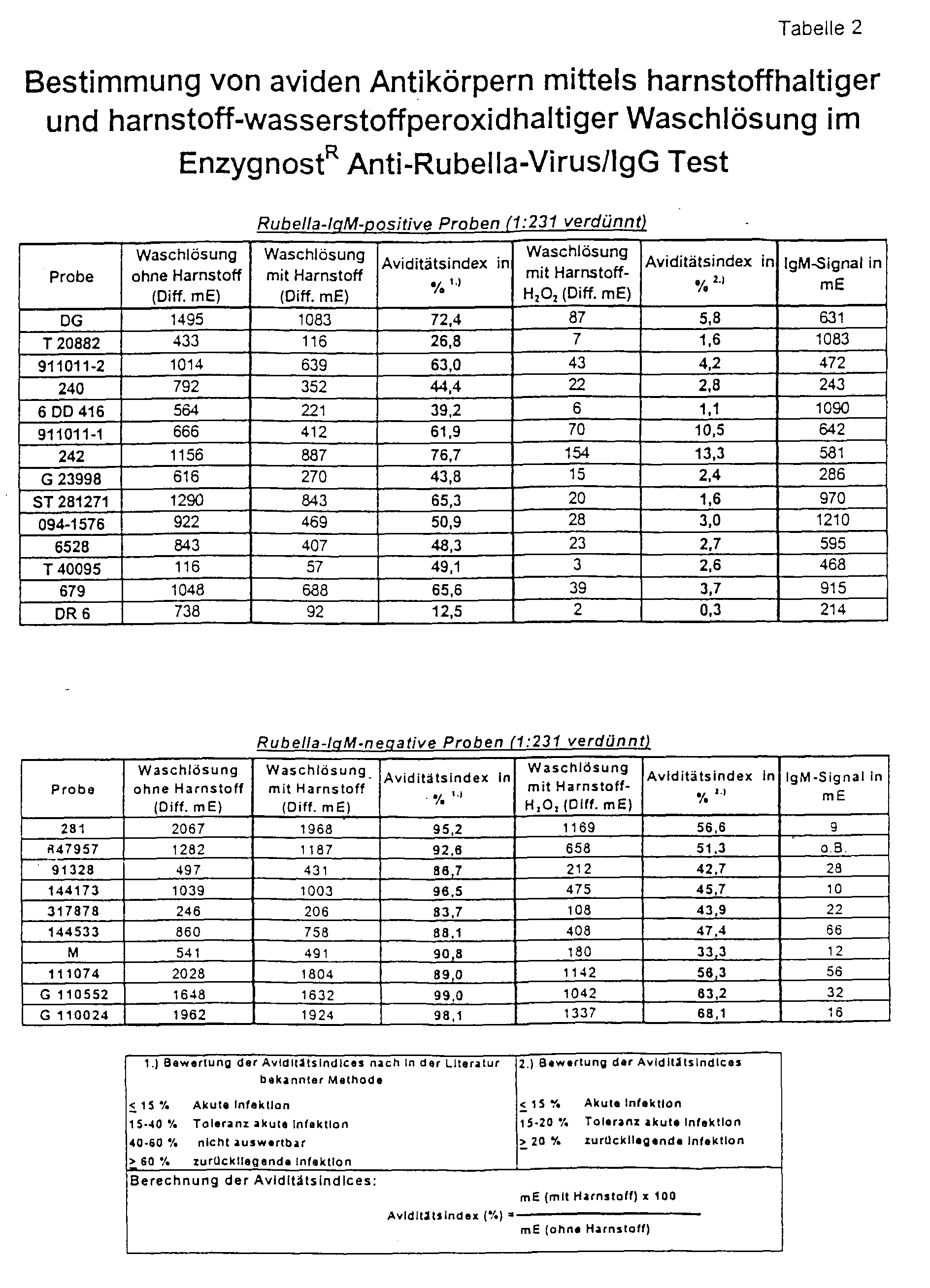

### Beispiel 4:

Zur Überprüfung des Beispiels 3 im System Röteln soll die Trennschärfe der Methode nochmals am Beispiel Herpes Simplex Virus (HSV) gezeigt werden. Es wird ein gesichert HSV-lgM positives Serumkollektiv mit einem gesichert HSV-lgM negativem Kollektiv verglichen.

### Ergebnis:

Siehe Tabelle 3. Die Hauptmenge der Seren aus der frühen Phase der Herpes Simplex Infektion erreicht einen Aviditätsindex von circa kleiner 5%, während Herpes Simplex Seren aus der Spätphase der Infektion einen durchschnittlichen Aviditätsindex von circa größer 50 % haben.

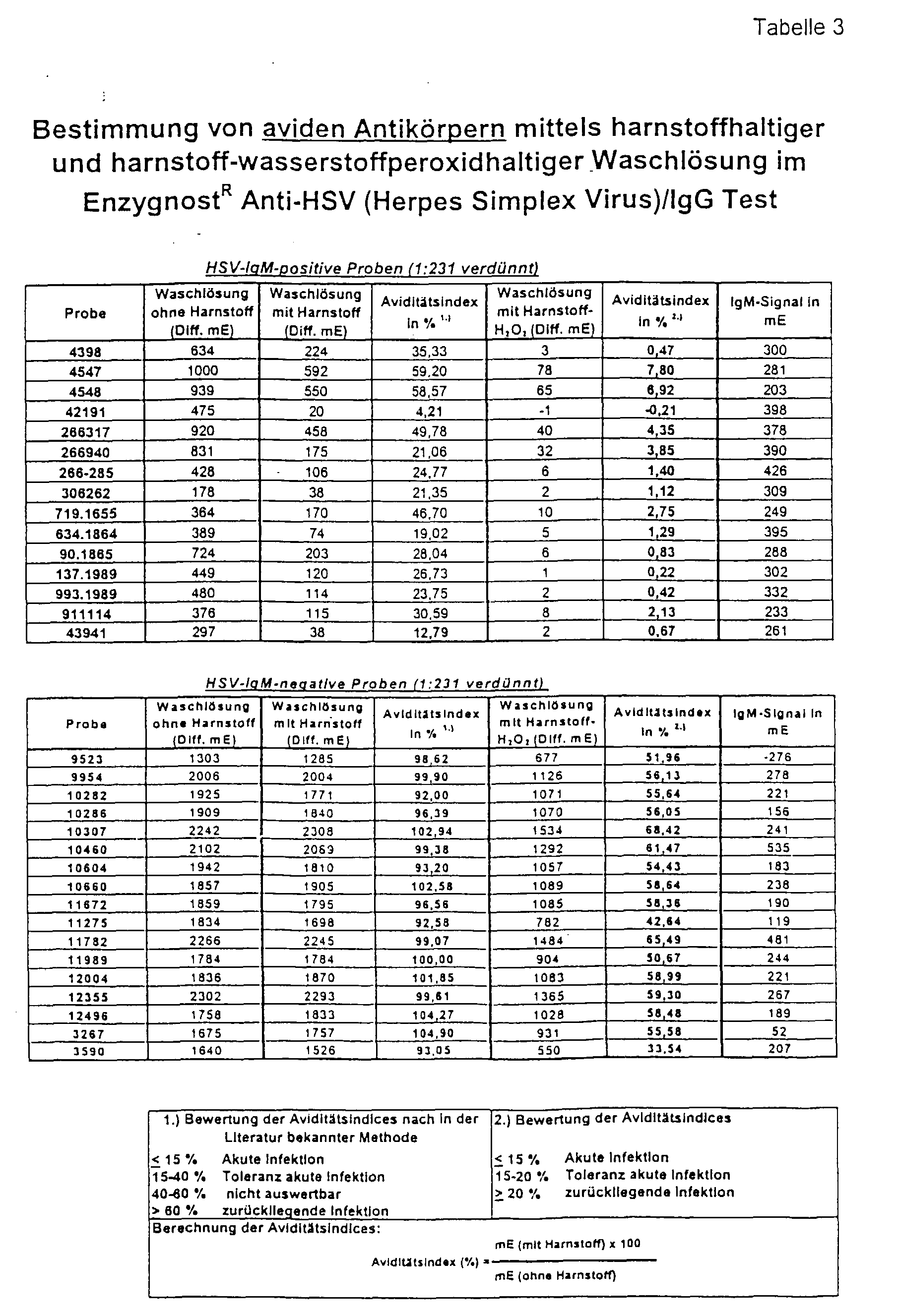

## Patentansprüche

1. Verfahren zum qualitativen oder quantitativen Nachweis eines Antikörpers, worin dieser Antikörper mit dem Antigen, gegen welches er gerichtet ist, in Kontakt gebracht wird, so daß sich Immunkomplexe bilden können, und worin der Reaktionsansatz mit einem proteindenaturierenden Agens in Kontakt gebracht wird, welches Immunkomplexe enthaltend Antikörper niedriger Avidität destabilisiert, während Immunkomplexe enthaltend Antikörper höherer Avidität weitgehend erhalten bleiben, und worin das Ausmaß der Bindung des Antikörpers mit dem Antigen bestimmt wird, dadurch gekennzeichnet, daß das proteindenaturierende Agens Harnstoff-Wasserstoffperoxid ist.

2. Verfahren zur Bestimmung der Avidität eines Antikörpers, worin der Antikörper in einem ersten und einem zweiten Ansatz unabhängig voneinander mit dem Antigen, gegen welches der Antikörper gerichtet ist, in Kontakt gebracht wird, so daß sich Immunkomplexe bilden können, und worin einer der beiden Ansätze mit einem proteindenaturierenden Agens in Kontakt gebracht wird, welches Immunkomplexe enthaltend Antikörper niedriger Avidität destabilisiert, während Immunkomplexe enthaltend Antikörper höherer Avidität weitgehend erhalten bleiben, und worin das Ausmaß der Bindung des Antikörpers mit dem Antigen in beiden Proben unabhängig voneinander nach bestimmt wird, und wobei sich die Avidität des Antikörpers aus dem Verhältnis des Ausmaßes der Antigen-Antikörperbindungen des ersten und des zweiten Ansatzes ergibt, dadurch gekennzeichnet, daß das proteindenaturierende Agens Harnstoff-Wasserstoffperoxid ist.

3. Verfahren gemäß Anspruch 1 oder 2, worin das Antigen in festphasengebundener Form mit dem Antikörper in Kontakt gebracht und anschließend mit einer harnstoff-wasserstoffperoxidhaltigen Pufferlösung gewaschen wird.

4. Verfahren gemäß einem der vorhergehenden Ansprüche, dadurch gekennzeichnet, daß es als Enzymimmuntest, Radioimmuntest, Western Blot oder Immunfluoreszenztest ausgeführt wird.

5. Verfahren gemäß einem der vorhergehenden Ansprüche, dadurch gekennzeichnet, daß es zum Nachweis diagnostisch relevanter Antikörper in Körperflüssigkeiten verwendet wird.

6. Verfahren gemäß Anspruch 5, dadurch gekennzeichnet, daß die diagnostisch relevanten Antikörper gegen Viren, Bakterien oder Parasiten gerichtet sind.

7. Verfahren gemäß Anspruch 5 oder 6, dadurch gekennzeichnet, daß die diagnostisch relevanten Antikörper gegen EBV, Rötelnvirus, CMV, Hantavirus, Parvovirus B19, VZV, HHV 6, HBV, HCV, HIV, RSV, HSV-1, HSV-2 oder Toxoplasma gondii gerichtet sind.

8. Verfahren gemäß einem der vorhergehenden Ansprüche, dadurch gekennzeichnet, daß Harnstoff-Wasserstoffperoxid in einer Konzentration von 2,5 bis 6,5 M eingesetzt wird.

9. Verfahren zur Differenzierung früher Infektionen von älteren Infektionen durch Bestimmung der Antikörperavidität nach einem Verfahren gemäß einem der vorhergehenden Ansprüche.

10. Verfahren gemäß einem der vorhergehenden Ansprüche zum Nachweis einer akuten oder kürzlich abgelaufenen Rötelnvirusinfektion, CMV-Erstinfektion, EBV-Erstinfektion, HSV-Erstinfektion oder Toxoplasma gondii-Infektion.

11. Waschlösung für Immunoassays, enthaltend Harnstoff-Wasserstoffperoxid.

12. Probenverdünnungspuffer für Immunoassays, enthaltend Harnstoff-Wasserstoffperoxid.

13. Waschlösung oder Probenverdünnungspuffer gemäß Anspruch 11 oder 12, worin Harnstoff-Wasserstoffperoxid in einer Konzentration von 2,5 bis 6,5 mol/l vorliegt.

14. Waschlösung oder Probenverdünnungspuffer gemäß Anspruch 13, worin Harnstoff-Wasserstoffperoxid in einer Konzentration von 4,5 bis 6,0 mol/l vorliegt.

15. Waschlösung oder Probenverdünnungspuffer gemäß Anspruch 13, worin Harnstoff-Wasserstoffperoxid in einer Konzentration von etwa 5,3 mol/l vorliegt.

16. Diagnostikum zur Durchführung eines Verfahrens gemäß einem der Ansprüche von 1 bis 10.

## Claims

1. A method for the qualitative or quantitative detection of an antibody, in which this antibody is brought into contact with the antigen against which it is directed so that immune complexes are able to form, and in which the reaction mixture is brought into contact with a protein-denaturing agent which destabilizes immune complexes containing antibodies of low avidity, while immune complexes containing antibodies of higher avidity are substantially retained, and in which the extent of the binding of the antibody to the antigen is determined, wherein the protein-denaturing agent is urea-hydrogen peroxide.

2. A method for determining the avidity of an antibody, in which the antibody is brought into contact in a first and a second mixture independently of one another with the antigen against which the antibody is directed so that immune complexes are able to form, and in which one of the two mixtures is brought into contact with a protein-denaturing agent which destabilizes immune complexes containing antibodies of low avidity, while immune complexes containing antibodies of higher avidity are substantially retained, and in which the extent of the binding of the antibody to the antigen in both samples is determined independently of one another, and where the avidity of the antibody is revealed by the ratio of the extent of the antigen-antibody bindings in the first and the second mixture, wherein the protein-denaturing agent is urea-hydrogen peroxide.

3. The method as claimed in claim 1 or 2, in which the antigen is brought into contact, in a form bound to a solid phase, with the antibody, and subsequently washed with a buffer solution containing urea-hydrogen peroxide.

4. The method as claimed in any of the preceding claims, which is carried out as enzyme immunoassay, radioimmunoassay, Western blot or immunofluorescence assay.

5. The method as claimed in any of the preceding claims, which is used for detecting diagnostically relevant antibodies in body fluids.

6. The method as claimed in claim 5, wherein the diagnostically relevant antibodies are directed against viruses, bacteria or parasites.

7. The method as claimed in claim 5 or 6, wherein the diagnostically relevant antibodies are directed against EBV, rubella virus, CMV, hantavirus, parvovirus B19, VZV, HHV 6, HBV, HCV, HIV, RSV, HSV-1, HSV-2 or Toxoplasma gondii.

8. The method as claimed in any of the preceding claims, wherein urea-hydrogen peroxide is employed in a concentration of from 2.5 to 6.5 M.

9. A method for differentiating early infections from older infections by determining the antibody avidity by a method as claimed in any of the preceding claims.

10. The method as claimed in any of the preceding claims for detecting an acute rubella virus infection, or one which has recently completed its course, a first CMV infection, first EBV infection, first HSV infection or Toxoplasma gondii infection.

11. A wash solution for immunoassays comprising urea-hydrogen peroxide.

12. A sample dilution buffer for immunoassays comprising urea-hydrogen peroxide.

13. A wash solution or sample dilution buffer as claimed in claim 11 or 12, in which urea-hydrogen peroxide is present in a concentration of from 2.5 to 6.5 mol/l.

14. A wash solution or sample dilution buffer as claimed in claim 13, in which urea-hydrogen peroxide is present in a concentration of from 4.5 to 6.0 mol/l.

15. A wash solution or sample dilution buffer as claimed in claim 13, in which urea-hydrogen peroxide is present in a concentration of about 5.3 mol/l.

16. A diagnostic aid for carrying out a method as claimed in any of claims 1 to 10.

## Revendications

1. Procédé pour la détermination qualitative ou quantitative d'un anticorps, dans lequel cet anticorps est mis en contact avec l'antigène contre lequel il est dirigé, de sorte que des complexes immuns peuvent se former, et dans lequel le mélange réactionnel est mis en contact avec un agent dénaturant les protéines, qui déstabilise les complexes immuns contenant des anticorps de faible avidité, tandis que les complexes immuns contenant des anticorps de plus forte avidité sont dans une large mesure conservés, et dans lequel le degré de la liaison de l'anticorps avec l'antigène est déterminé, caractérisé en ce que l'agent dénaturant les protéines est l'urée-peroxyde d'hydrogène.

2. Procédé pour la détermination de l'avidité d'un anticorps, dans lequel l'anticorps, dans un premier mélange de départ et un second mélange de départ, indépendamment l'un de l'autre, est mis en contact avec l'antigène contre lequel est dirigé l'anticorps, de sorte que des complexes immuns peuvent se former, et dans lequel l'un des deux mélanges est mis en contact avec un agent dénaturant les protéines, qui déstabilise les complexes immuns contenant des anticorps de faible avidité, tandis que les complexes immuns contenant des anticorps de plus forte avidité sont dans une large mesure conservés, et dans lequel le degré de la liaison de l'anticorps avec l'antigène dans les deux échantillons, indépendamment l'un de l'autre, est déterminé, et l'avidité de l'anticorps résulte du rapport des degrés des liaisons antigène-anticorps du premier mélange et du second, caractérisé en ce que l'agent dénaturant les protéines est l'urée-peroxyde d'hydrogène.

3. Procédé selon la revendication 1 ou 2, dans lequel l'antigène, sous forme fixée à une phase solide, est mis en contact avec l'anticorps, et est ensuite lavé avec une solution tampon contenant de l'urée-peroxyde d'hydrogène.

4. Procédé selon l'une des revendications précédentes, caractérisé en ce qu'il est effectué en tant qu'essai immunoenzymatique, essai radio-immunologique, analyse western blot ou essai par fluorescence.

5. Procédé selon l'une des revendications précédentes, caractérisé en ce qu'il est utilisé pour la détection d'anticorps d'intérêt pour le diagnostic, dans des liquides corporels.

6. Procédé selon la revendication 5, caractérisé en ce que les anticorps d'intérêt pour le diagnostic sont dirigés contre des virus, des bactéries ou des parasites.

7. Procédé selon la revendication 5 ou 6, caractérisé en ce que les anticorps d'intérêt pour le diagnostic sont dirigés contre EBV, le virus de la rubéole, CMV, l'hantavirus, le parvovirus B19, VZV, HHV-6, HBV, HCV, HIV, RSV, HSV-1, HSV-2 ou *Toxoplasma gondii.*

8. Procédé selon l'une des revendications précédentes, caractérisé en ce que l'urée-peroxyde d'hydrogène est utilisé à une concentration de 2,5 à 6,5 M.

9. Procédé pour la distinction d'infections précoces d'avec des infections plus anciennes, par détermination de l'avidité d'anticorps, conformément à un procédé selon l'une des revendications précédentes.

10. Procédé selon l'une des revendications précédentes, pour la détection d'une infection à virus de la rubéole aiguë ou ayant eu lieu depuis peu, une primo-infection à CMV, une primo-infection à EBV, une primo-infection à HSV ou une infection à *Toxoplasma gondii.*

11. Solution de lavage pour dosages immunologiques, contenant de l'urée-peroxyde d'hydrogène.

12. Tampon pour dilution d'échantillons pour dosages immunologiques, contenant de l'urée-peroxyde d'hydrogène.

13. Solution de lavage ou tampon pour dilution d'échantillons selon la revendication 11 ou 12, dans lesquels l'urée-peroxyde d'hydrogène est présent à une concentration de 2,5 à 6,5 moles/l.

14. Solution de lavage ou tampon pour dilution d'échantillons selon la revendication 13, dans lesquels l'urée-peroxyde d'hydrogène est présent à une concentration de 4,5 à 6,0 moles/l.

15. Solution de lavage ou tampon pour dilution d'échantillons selon la revendication 13, dans lesquels l'urée-peroxyde d'hydrogène est présent à une concentration d'environ 5,3 moles/l.

16. Agent de diagnostic pour la mise en oeuvre d'un procédé selon l'une des revendications 1 à 10.
